# EUROPEAN PATENT APPLICATION

(11) **EP 0 628 322 A2**
(43) Date of publication of application: **14.12.1994**
(21) Application number: 94201598.3
(22) Date of filing: 06.06.1994
(51) Int. Cl.: A61M 25/01, A61B 17/38

(54) **Flexible catheter with strip-like electrode**

(30) Priority: 11.06.1993 NL 9301018
(71) Applicant: CORDIS EUROPA N.V., NL-9301 LJ Roden (NL)
(72) Inventor: Van Erp, Wilhelmus Martinus Maria, NL-9351 KS Leek (NL)
(74) Representative: 't Jong, Bastiaan Jacobus

(57) **Abstract**

The invention relates to a catheter comprising a tube-like basic body with a distal end and a proximal end provided with a connecting means, at least one continuous channel and with a flexible part at the distal end.
In the flexible end-section a pull wire, extending through the channel to the outside of the proximal end, is attached so as to bend the end-section by pulling the pull wire. A bendable strip-like electrode is formed by a lateral surface portion of a wound wire, received in a depression in the end section.

## Description

The invention relates to a controlled steerable catheter. A guiding catheter with a controllable distal tip is known from EP-A-0 361 314.

The invention as characterized in claim 1 provides a controlled flexible intervential catheter with a strip-like electrode at its distal end. A catheter of this kind is for instance used for treating certain types of cardiac arrhythmias.

With the catheter according to the invention the strip-like electrode can be positioned accurately against the wall of for instance a ventricle, in order to locally heat and ablate tissue when treating those types of cardiac arrythmias. The electrode does not hinder the flexing of the end part.

The invention will be explained in greater detail in the following description with reference to the examples of the embodiments as shown in the drawings.
- **Fig. 1**: represents a catheter according to the invention in the position of use.
- **Fig. 2**: shows the end-section of a catheter according to a first embodiment of the invention in a partly longitudinal cross-sectional view.
- **Fig. 3**: shows a section according to III-III in fig. 2 on a larger scale.
- **Fig. 4**: is a partly schematic view of a catheter according to a second embodiment.
- **Fig. 5**: shows a cross-section at V-V in fig. 4.
- **Fig. 6**: shows a cross-section at VI-VI in fig. 4.

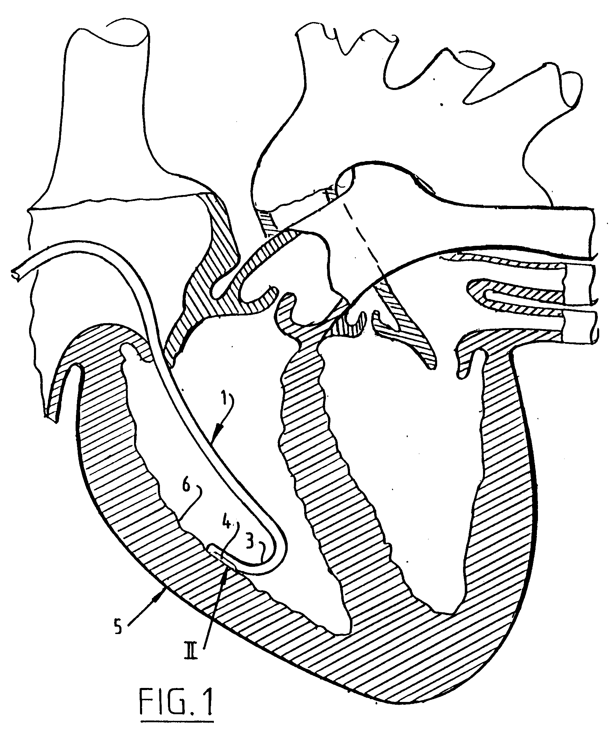

As fig. 1 shows, a catheter 1 according to the invention can be advanced in the usual manner through a vein to the right ventricle in order to be positioned firmly against the wall 6 of the right ventricle of the heart 5.

The catheter 1 comprises a basic body with a proximal end, in the usual manner provided with a connecting piece not shown here remaining outside the body, and a distal end comprising a flexible end-section 3.

With this embodiment the wall of the flexible end-section is provided with a strip-like electrode extending in the longitudinal direction of the catheter, such as a heating element.

The catheter 1 is manufactured in such a way that its end-section 3 can be bend reliably in a controlled fashion in order to place electrode 4 firmly against the wall 6. When the electrode 4 is a heating element, as described above, a certain section of the wall 6 can be heated in this way and consequently the tissue disturbed when treating certain types of cardiac arrhythmias.

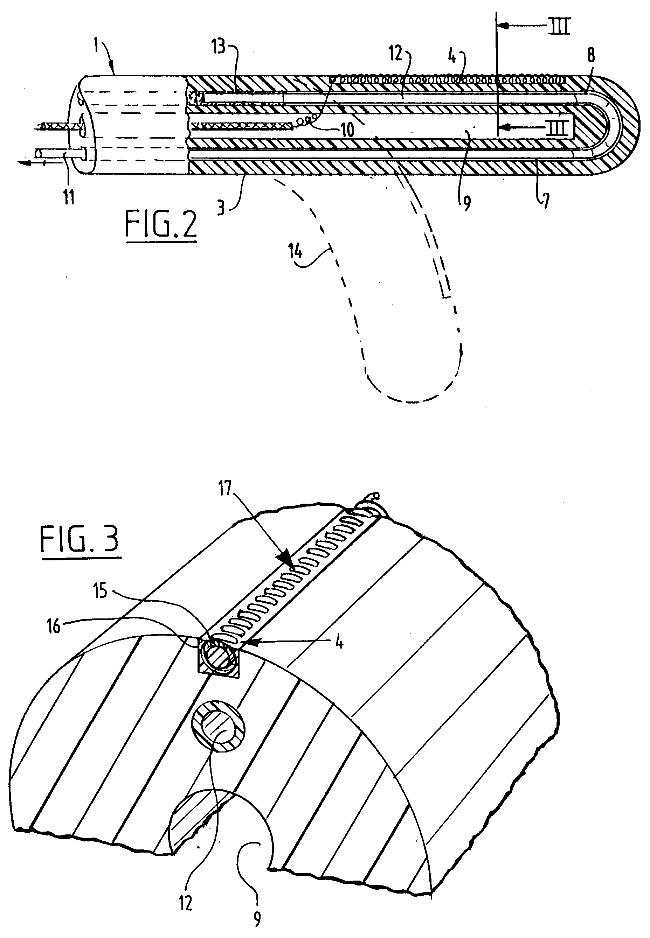

As fig. 2 shows in greater detail, the catheter 1 is provided with an eccentrically positioned channel 7, in which a pull wire 11 has been incorporated. This pull wire has been fixed eccentrically in the end-section 3, by incorporating a doubled up end-piece 12 of the pull wire 11 in a cavity 8. The end-piece 12 can be placed against an end wall of the body of the catheter, at the position of the connection with a separate flexible end-section.

The pull wire 11 extends over the full length of the catheter 1 through the channel 7 and ends, close to the proximal end outside the catheter. By pulling pull wire 11, the end-section 3 of the catheter 1 bends in the direction of channel 7, in which the pull wire 11 has been incorporated. The deflection has been illustrated schematically and is indicated with reference numberal 14.

The doubled up end-piece 12 of the pull wire 11 has been secured in channel 8 with glue 13. Another possibility being, that the end-section 3 is a separate end-section of the catheter 1 and that the doubled back end-piece 12 of the pull wire is placed against the end wall of the basic body at the position of the connection between the end-section and the basic body.

Fig. 2 also shows cross-sectionally the strip-like electrode executed as heating element 4 together with a connecting wire 10, running through a central channel 9 in the catheter, to the proximal end where this connecting wire is connected.

In relation to the channel 7, the electrode 4 is placed at the opposite side of the catheter, so that this electrode 4 is always positioned on the outside curve of the bent catheter and consequently can be moved firmly against the desired object.

It is obviously also possible to place the electrode at the opposite side, close to the eccentrically located channel 7 in which case the electrode will always be placed on the inside of the curve.

Fig. 3 shows more in detail that the strip-like electrode 4 according to the invention, consists of a helically wound wire 15 incorporated in a depression 16 in the end section. The depression 16 has a depth somewhat smaller than the diameter of the wound wire 15. Accordingly, a lateral surface portion 17 is exposed at the outside of the catheter and forms the active surface of the electrode. The helically wound wire 15 is fixed in the depression 16 by means of a suitable filling adhesive.

In another embodiment the depression can have a depth equal to the diameter of the wound wire. After embedding the wound wire in the depression 16 the outer surface of the end section of the catheter is ground or polished, so as to expose the lateral surface portion of the wound wire.

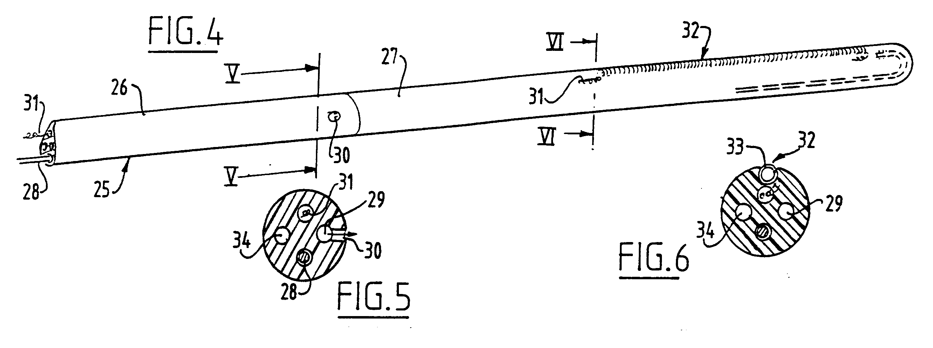

The catheter 25, partly illustrated in fig. 4, is also made up of a basic body 26 and a flexible end-section 27. As fig. 5 and 6 show, both in the basic body 26 and the end-section 27, four channels have been formed. The bottom channel in fig. 5 and 6 comprises a pull wire 28 which is, as described above, fixed eccentrically with a doubled up end-piece in the end-section close to the tip.

In this embodiment the channel 29 is used for the supply of contrast medium close to the tip of the catheter, so that the area surrounding the catheter can be made visible on an X-ray screen in a catheterization laboratory. The contrast substance conveyed can be delivered through holes such as hole 30.

The end-section 27 is in this case provided with a strip-like electrode 32, which has been formed by the lateral surface 33 of a helically wound wire embedded in the material making up the basic body. Connecting wire 31 has been fed to the proximal end through the upper channel.

By manufacturing the electrode 32 in the form of a helically wound wire, the latter is very flexible and also to some extent elastic, thus not affecting the flexibility of the end-section 27 adversely.

The fourth channel 34 in the illustrated example of an embodiment, can be used for a guide wire.

The invention is not restricted to embodiments in which the electrode is formed by a helically wound wire in a groove-like depression in the catheter wall. The electrode can also be wound zigzag and be taken up in a depression in the surface of the catheter, which depression has a depth not higher than the diameter of the wire.

Furthermore the helically wound wire can extend coaxially around the end-section of the catheter, the depression in that case forming a part of the basic body having a smaller diameter.

## Claims

1. Catheter comprising a tube-like basic body with a distal end and a proximal end provided with a connecting means, at least one continuous channel and with a flexible part at the distal end, wherein means for bending the flexible end section are attached in the flexible end-section, the wall of the flexible end-section comprising a strip-like electrode extending in the longitudinal direction of the catheter, an excitation conductor for the heating element running from the electrode to the proximal end, said strip-like electrode consisting of a wound wire incorporated in a depression in the end-section.

2. Catheter according to claim 1, wherein the depression in the end-section has the form of a groove and has a depth at highest equal to the diameter of the wound wire.

3. Catheter according to claim 2, wherein the wire is wound zigzag-like.

4. Catheter according to claim 1, wherein the wire is wound helically and extends coaxially around the catheter in a part with smaller diameter forming the depression.
